# EUROPEAN PATENT SPECIFICATION

(11) **EP 2 282 639 B1**
(45) Date of publication and mention of the grant of the patent: **03.12.2014**
(21) Application number: 09749917.2
(22) Date of filing: 22.05.2009
(51) Int. Cl.: A01N 63/00

(54) **INCREASING THE SHELF LIFE OF BAKERY AND PATISSERIE PRODUCTS BY USING THE ANTIFUNGAL LACTOBACILLUS AMYLOVORUS DSM 19280**
VERLÄGERUNG DER HALTBARKEIT VON BACK- UND KONDITORWAREN MIT DEM ANTIMYKOTISCHEN LACTOBACILLUS AMYLOVORUS DSM 19280
AUGMENTATION DE LA DURÉE DE VIE EN RAYON DE PRODUITS DE BOULANGERIE ET DE PÂTISSERIE À L'AIDE DE LACTOBACILLUS AMYLOVORUS ANTIFONGIQUE DSM 19280

(30) Priority: 22.05.2008 IE 20080405
(43) Date of publication of application: 16.02.2011
(73) Proprietor: University College Cork-National University of Ireland, Cork, Cork (IE)
(72) Inventor: ARENDT, Elke K, Wilton Cork (IE); DAL BELLO, Fabio, Wilton Cork (IE); RYAN, Liam, Wilton Cork (IE)
(74) Representative: Purdylucey Intellectual Property
(86) International application number: PCT/EP2009/056229
(87) International publication number: WO 2009/141427

(56) References cited:
- DAL BELLO ET AL: "Improvement of the quality and shelf life of wheat bread by fermentation with the antifungal strain Lactobacillus plantarum FST 1.7" JOURNAL OF CEREAL SCIENCE, ACADEMIC PRESS LTD, XX, vol. 45, no. 3, 13 April 2007 (2007-04-13) , pages 309-318, XP022021096 ISSN: 0733-5210
- CHENG S S ET AL: "Antifungal activity of cinnamaldehyde and eugenol congeners against wood-rot fungi" BIORESOURCE TECHNOLOGY, ELSEVIER BV, GB, vol. 99, no. 11, 1 July 2008 (2008-07-01), pages 5145-5149, XP022606336 ISSN: 0960-8524 [retrieved on 2007-10-22]
- DE MUYNCK C ET AL: "Potential of selected lactic acid bacteria to produce food compatible antifungal metabolites" MICROBIOLOGICAL RESEARCH, FISCHER, JENA, DE, vol. 159, no. 4, 15 December 2004 (2004-12-15), pages 339-346, XP004956710 ISSN: 0944-5013

## Description

### Field of the Invention

The present invention relates to a novel strain of *Lactobacillus amylovorus,* which has antifungal properties and the ability to prolong the shelf-life of food products such as yeast or chemically leavened cereal products. Examples of baked products include but are not limited to breads and cakes, and intermediate products such as doughs, batters or sourdoughs. The invention also relates to the antifungal fermentation broth or extract of this strain as well as newly identified antifungal compounds produced by the strain.

### Background to the Invention

Bread is the most important staple food in the Western world and it is generally viewed as a perishable commodity, which is best consumed when 'fresh'. The loss of perceived freshness is due to a number of factors, which may generally be categorised into one of two groups: those that are due to a series of complex processes collectively known as staling; and those that are attributed to microbial spoilage. Despite being studied for more than a century and a half, bread staling has not been eliminated and remains responsible for huge economic losses to both the baking industry and the consumer (Gray and Bemiller, 2003). The application of lactic acid bacteria (LAB) in the form of sourdough has been reported to have positive effects on wheat bread quality and staling (Corsetti et al., 2000; Clarke et al., 2002; Crowley et al., 2002).

The most frequent cause of microbial spoilage in bread is mould growth. Common spoilage fungi from bakery products belong to the genera *Penicillium, Aspergillus, Monilia, Mucor, Endomyces, Cladosporium, Fusarium* and *Rhizopus* (Legan, 1993; Poute and Tsen, 1987). In particular, *Penicillium roqueforti* is highly resistant to antifungal compounds, and it is responsible for about 70% of bread spoilage. In addition to the economic losses associated with spoilage of this nature, a further concern is the possibility that mycotoxins produced by the moulds may cause public health problems (Legan, 1993). A number of methods are applied to prevent or minimise microbial spoilage of bread, e.g. addition of propionic acid and its salts, modified atmosphere packaging, irradiation, pasteurisation of packaged bread (Legan, 1993; Pateras, 1998), or biopreservation (i.e. control of one organism by another). The recent years have experienced an increasing interest in the application of biopreservation in the food industry. In this regard, LAB are of special interest, since they have a long history of use in food and, in particular, lactobacilli are 'generally regarded as safe'. Beside the weak organic acids, i.e. lactic and acetic acids (Röcken and Voysey, 1995; Röcken, 1996; Stiles, 1996), LAB produce a wide range of low molecular weight compounds (Niku-Paavola et al., 1999), peptides (Okkers et al., 1999) and proteins (Magnusson and Schnürer, 2001) with antifungal activity. Recently, the production of the antifungal cyclic dipeptides cyclo (_{L}-Phe-_{L}-Pro) and cyclo (_{L}-Phe-*trans*-4-OH-_{L}-Pro) has been shown for *Lactobacillus plantarum* MiLAB 393 (Ström et al., 2002). This strain was found to inhibit the growth of *Aspergillus nidulans* and to alter the fungal protein expression during co-cultivation studies (Ström et al., 2005). Cyclic dipeptides have been previously shown to be both antibacterial and antifungal (Graz et al., 1999) and it is likely that these substances, previously only reported from *L. plantarum* strains (Lindgren and Dobrogosz, 1990), are also produced by other LAB, e.g. *Pediococcus pentosaceus* and *Lactobacillus sakei* (Magnusson et al., 2003). Unfortunately, most of these investigations rely mainly on studies using laboratory media, which, even if suitable for testing activity, may however not reflect the situation encountered in a food system. Furthermore, during these studies some of the antifungal strains were found to lose their activity over time (Magnusson et al., 2003). Finally, the applicability of these antifungal strains as starters for fermentations has not always been considered nor has the quality of the final product been described.

*Lactobacillus amylovorus* has been noted as one of the dominant strains in type II sourdoughs and inhibitory substances produced by lactobacilli isolated from sourdoughs have been identified. The potential of selected lactic acid bacteria to produce food-compatible antifungal metabolites has been recorded. US patent 6,827,952 describes *Lactobacillus sanfranciscensis* strains with mould-proofing activity and a method for producing bread. De Muynck et al. (2004) reported the isolation of 13 antifungal strains of LAB, one of which is recorded as *Lactobacillus amylovorus.* Corsetti et al. (2000) describe the addition of a *L. amylovorus* strain to sourdough and the positive effects of its amylolytic activity on bread firmness and staling. The delay of onset of fungal growth by 7 days in bread started with *Saccharomyces cerevisiae* and the sourdough isolate *L. plantarum* 21B has been reported. A notable property of antifungal activity demonstrable by bacterial strains is that it is often strain specific, affecting individual strains of target organisms and not others of the same species, thus providing a requirement for a number of producer strains in order to demonstrate a wide inhibitory spectrum. Other antifungal moieties (plant derived, essential oils, fatty acids, modified whey) are known but would not be suitable for use as starter cultures.

### Object of the Invention

The object of this invention is to isolate and characterise lactic acid bacteria with antifungal properties. A further object is to provide antifungal strains as starters for sourdough fermentation, and apply them in food preparation, more specifically in the production of bread and baked cereal products. Sourdough fermented by the antifungal *L. amylovorus* will be compared to sourdough fermented by traditional sourdough isolates, e.g. *Lactobacillus plantarum* and/or *Lactobacillus sanfranciscensis,* as well as to a chemically acidified dough and a non-acidified dough. Another object is to provide use of the strains to reduce staling of bread and baked cereal products. A still further object is to provide antifungal compounds responsible for the activity of the strains. Another object is to provide antifungal strains and extracts thereof for use in food preservation (e.g. for cereal, dairy, or meat products, as well as fruit and vegetables), as preservatives for animal feed production (e.g. silage), treatment of surfaces (e.g. wood) and pharmaceutical compositions for human and animal use (e.g. disinfectants, creams).

### Summary of the Invention

In a first aspect, the invention provides a strain of *Lactobacillus amylovorus* designated FST 2.11 as deposited under the accession no DSM 19280 on 13^{th} April 2007 in the DSMZ depository.

The invention also provides a starter culture for bread including low salt bread, or cereal-based products comprising a strain of the invention.

The invention also provides a food ingredient comprising a strain of the invention.

The invention also provides an antifungal agent active against spoilage moulds, comprising a strain of the invention.

The invention also provides a method of production of fermented cereal products or breads including low salt bread, comprising addition of the strain of the invention to the cereals or bread starting materials.

The invention also provides a pharmaceutical composition comprising a strain of the invention and a pharmaceutically acceptable carrier or excipient.

### Brief Description of the Drawings

**Figure 1**. Partial 16S rDNA sequence of the strain of LAB designated FST 2.11. To determine the closest relatives of 16S rDNA sequences, a search of the GenBank DNA database was conducted by using the BLAST algorithm.
**Figure 2**. HPLC profile of water soluble (A), 10% acetonitrile soluble (B) and 50% acetonitrile soluble(C) compounds produced by *L. amylovorus* FST 2.11. Compounds indicated with an arrow showed antifungal activity.
**Figure 3**. Microtiter well spore germination bioassay system used to identify antifungal compounds produced by *L. amylovorus* FST 2.11. The figure shows the compounds present in the 50% acetonitrile soluble fraction.
**Figure 4**. LAB cell counts (A), pH (B) and TTA (C) values of wheat sourdoughs fermented by *L. amylovorus* FST 2.11 (red line), *L. plantarum* FST 1.7 (black line) or *L. sanfranciscensis* LTH 2581 (green line) for 48 h at 30°C. LAB total cell counts were determined on mMRS4 agar.
**Figure 5****.** Shelf life of wheat bread and wheat bread containing 20% sourdough fermented for 24 or 48 h. Bread slices were sprayed with about 10⁴ spores of *A*. *niger, F. culmorum, P. expansum* or *P. roqueforti,* stored at room temperature and mould growth was monitored over 10 days.
**Figure 6**. Specific volume (A), moisture content (B), total volume of CO₂ (C), CO₂ released (D), and CO₂ retained in wheat bread (CON), bread containing a chemically acidified dough (CA) and sourdough bread fermented by *L. plantarum* (LP), *L. sanfranciscensis* (LS), or *L. amylovorus* (LA).
**Figure 7****.** Hardness (A), rate of staling (B), and gumminess (C) of wheat bread (CON), bread containing a chemically acidified dough (CA) and sourdough wheat bread fermented by *L. sanfranciscensis* (LS), *L. plantarum* (LP) or *L. amylovorus* (LA) over 5 days of storage at room temperature.
**Figure 8****.** Shelf life of lean bread containing 40% (20% flour replacement) sourdough inoculated with *Lactobacillus amylovorous.* (Sourdough preparation:50% wheat flour

Inoculum : *Lactobacillus amylovorus* FST 2.1 : 10⁵ CFU/g dough.Conditions : 30°C and 48h), Lean Bread : 5% Fresh yeast, 2% salt.

### Detailed Description of the Invention

### Isolation and identification of sourdough LAB

Gluten-free sourdough was prepared according to a recipe recently developed at the UCC (Table I). Fermentation was performed either at 30°C or 37°C. LAB from 24 h-fermented gluten-free sourdoughs were cultivated on mMRS5 (Meroth et al., 2003) agar supplemented with 0.05g/l Bromcresol green and incubated at 30°C for 48 h under anaerobic conditions. From the plates containing 30 to 300 colonies, 3 colonies per colony form were subcultured and identified by sequence analysis of the first 1000 base pairs (bp) of the 16S rDNA. To determine the closest relatives of the partial 16S rDNA sequences, a GenBank DNA database search was conducted. A similarity of > 98% to 16S rDNA sequences of type strains was used as the criterion for identification.

### Fungal cultures and preparation of the spore solution

The moulds *Aspergillus fumigatus* J9, *Aspergillus niger* FST 4.21, *Fusarium culmorum* FST 4.05, *Fusarium graminearum* FST 4.02, *Penicillium expansum* FST 4.22 as well as *Penicillium roqueforti* FST 4.11 were used as target organisms for assay of antifungal activity *in vitro* (Table II). Moulds were cultivated on malt extract agar (Oxoid, Hampshire, UK) and the fungal spore solutions were obtained as described previously (Magnusson and Schnürer, 2001). Briefly, *A. niger, F. culmorum, P. expansum* and *P. roqueforti* were grown on malt extract agar until sporulation occurred. Spores were harvested in physiological solution and stored at -80°C in a glycerol/water (50:50 v/v) solution. Spores were transferred from this stock solution into a synthetic nutrient-poor medium (Nirenberg, 1976). Vigorous stirring (200 rpm) for 8 days at room temperature provided a fungal cell and conidial suspensions with a concentration of 5 × 10⁷ spores ml⁻¹.

### In vitro antimicrobial activity

The inhibitory activity of *L. amylovorus* FST 2.11 against selected spoilage moulds and bacteria (see Table II for full list) was investigated using the overlay method (Magnusson and Schnürer, 2001) with some modifications. To avoid any pH effect, the screening was carried out on buffered mMRS5 agar plates. The medium was buffered to pH 6.5 using a 75 mmol KH₂PO₄ solution. *Lactobacillus plantarum* FST 1.7 was included as a positive control, as the antifungal activity of this strain has been recently characterised (Dal Bello et al., 2007). Additionally, *Lactobacillus sanfranciscensis* LTH 2581 was used as a negative control. LAB strains were placed as cell spots on the plates and incubated at 30°C for 48 h in anaerobic jars. To test antimicrobial activity against spoilage bacteria, plates were overlaid with 12 ml of standard I agar (Merck, Germany) containing 100 µl of an overnight spoilage bacterial culture. Plates were then incubated at 30 or 37°C for 60 h. To investigate antifungal activity, a fungal spore solution (ca. 10⁴ spores / ml) was sprayed by nebulisation on the surface of plates. Plates were then incubated at room temperature for 3 days. Inhibitory activity was scored as follows: -, no inhibition; +, very weak inhibition around the colonies; ++, low inhibition with little clear zones around the colonies; +++, strong inhibition with detectable zones around the colonies; ++++, very strong inhibition with large clear zones and nearly no growth around the colonies.

### Isolation and characterisation of antifungal compounds produced by L. amylovorus FST 2.11

Antifungal compounds of *L. amylovorus* FST 2.11 were isolated according to the method of Ström et al. (2002) with a number of modifications. Briefly, a microtiter well spore germination bioassay was utilised to determine the activity of compounds from culture filtrate against the indicator fungus *A. fumigatus* J9. Cell-free supernatant was fractioned on a C¹⁸ solid phase extraction (SPE) column, allowing the separation of the hydrophilic phase from the hydrophobic phase. Prior to solid phase extraction a 10 ml sample of broth was taken and distilled with the distillate being examined for the presence of organic acids (up to 10 C-atoms). These acids were identified using comparative gas chromatography (GC) with mass spectroscopy (MS) with respect to elution time and molecular weight. All other compounds in the broth were separated using HPLC. To increase the ease of collection of compounds an initial separation was carried out with respect to elution time on the HPLC unit with the 99.8% MeCN phase being separated into 3 individual phases and evaluated separately. During separation all HPLC peaks corresponding to individual compounds were collected separately (no mass fractionating and bioassaying were performed) and once collected each compounds was either freeze dried or dried under a flow of clean dry nitrogen gas. Each compound was then evaluated for antimicrobial activity at a 50 mg/ml level using the spore germination bioassay described previously, with the level of outgrowth being examined both visually and also using a microtiter plate reader at a wave length of 490 nm. The chemical structure, molecular weight and fragmentation behaviour of compounds active in the bioassay were identified using ¹H nuclear magnetic resonance (NMR), LC-MS, and GC-MS. A Q TOF LC-MS was used to determine the molecular weights and fragmentation pattern of each compound (Table III), with these being compared to previously reported results. The collected ¹H NMR results were compared with previously reported NMR chemical shifts from a number of NMR databases. These results acted as a final confirmation of the molecule isolated.

### Sourdough fermentation and analysis

The suitability of *L. amylovorus* FST 2.11 as starter for wheat sourdough fermentation was investigated and compared to that of traditional sourdough starters *L. sanfranciscensis* LTH 2851 and *L. plantarum* FST 1.7. Briefly, 80 ml of mMRS5 broth were inoculated (1% level) with an overnight culture and incubated for 24 h at 30°C. Cells were harvested by centrifugation at 4000 rpm for 10 min, washed twice and resuspended in 40 ml sterile tap water (containing ca. 5 x 10⁹ CFU/ml). Six-hundred grams of wheat flour and 600 ml of sterile tap water (dough yield of 200) were mixed to homogeneity for 1 min with a Kenwood mixer mixed. The selected starter was inoculated at a final concentration of ca. 10⁵ CFU/g dough. Sourdough fermentation was performed at 30°C for 48 h. LAB cell growth during sourdough fermentation was investigated. Briefly, 1 g sourdough was serially diluted in sterile physiological solution and the LAB cell counts were determined on mMRS5 agar plates. At each time point, pH and TTA values were also measured using a suspension of sourdough (10 g), acetone (5 ml) and distilled water (95 ml) according to a standard method (Arbeitsgemeinschaft Getreideforschung e.V., 1994). To confirm the presence of the inoculated starter LAB, at the end of fermentation 3 colonies per colony form were picked from mMRS5 agar plates containing 30 to 300 colonies, purified and subjected to partial 16S rDNA sequencing according to a previously described method (Meroth et al., 2003).

### Sourdough bread production

The sourdoughs fermented by the selected LAB were used for the production of wheat bread. Doughs were prepared by replacing 20% of the flour with an equivalent quantity of flour in the form of sourdough fermented by the selected strain. Dough formulations based on a flour quantity of 3000 g were mixed in a Stephan mixer (Stephan Sohne, Hameln, Germany) at level 2 (1400 rpm) for 20 s prior to scraping down and mixed for further 40 s. The doughs were rested in bulk for 30 min in the proofer (Koma BV Roermond, Holland) at 30°C and 85% rh, scaled into 400 g portions, moulded in a small scale moulder (Machinefabriek Holtkamp BV, Almelo, Holland), placed in tins (180 mm x 120 mm x 60 mm, Sasa UK, Middx, UK) and proofed for 50 min at 30°C and 85% rh. Baking was carried out at 230°C for 30 min in a deck oven (MIWE, Arnstein, Germany). The oven was presteamed (300 ml of water) before loading and, on loading, was steamed by injecting 700 ml of water. The loaves were depanned and kept for 120 min on cooling racks at room temperature. Loaves were heat sealed in moisture impermeable bags (Polystyrol -Ethylene Vinyl Alcohol- Polyethylene) under modified atmosphere (60% N₂ and 40% CO₂) and stored at 21°C. Analyses were performed over a five-day storage period at three intervals: 2 h (after cooling and before packing), 50 h and 122 h, respectively. Additionally, a non-acidified dough as well as a chemically acidified dough were prepared. The chemically acidified dough contained a mixture of lactic and acetic acids (4:1 v/v) in order to yield a dough pH comparable to that of the doughs containing sourdough (biologically acidified).

### Bread analysis

A series of bread analysis were performed (in triplicate) prior to packaging. Loaf weight and volume (rapeseed displacement method) were determined. Bake loss and loaf specific volume (ml/g) were calculated. Crust and crumb colour were determined with a chroma-meter (Minolta CR-300, Osaka, Japan). For crumb texture analysis, loaves were sliced transversely using a slice regulator and bread knife to obtain uniform slices of 25 mm thickness. Two bread slices taken from the centre of each loaf were used. Images of the bread were captured using a flatbed scanner (HP ScanJet4c, Hewlett Packard) and supporting software (Desk Scan II, Hewlett Packard). The brightness levels were adjusted to 150 units and contrast to 170 units using software controls (Crowley et al., 2000). Texture profile analysis (TPA) was performed using a universal testing machine TA-XT2i (Stable Micro Systems, Surrey, UK) equipped with a 25-kg load cell and a 35 nun aluminium cylindrical probe. The settings used were a test speed of 2.0 mm/sec with a trigger force of 20 g to compress the middle of the breadcrumb to 60% of its original height. Water activity was determined with material taken from the centre of the crumb using the Aqua lab CX-2 (Decagon Devices Inc., Washington, USA). All measurements obtained with the three loaves from one batch were averaged into one value, i.e. one replicate. TPA was repeated with three loaves at day 2 (50 hr after baking) and day 5 (122 hr after baking).

### Bread challenge tests

Sourdoughs were fermented for 24 or 48 h at 30°C with the antifungal *L. amylovorus* FST 2.11 or *L. plantarum* FST 1.7, as well as with the control strain *L. sanfranciscensis* LTH 2581. Additionally, bread containing spontaneously fermented sourdough as well as a non-fermented bread and a chemically acidified bread were included. The antifungal activity of the LAB in the context of bread was determined using bread slices challenged against *A. niger, F. culmorum, P. expansum* as well as *P. roqueforti* spores according to previously described methods (Dal Bello et al., 2007). Briefly, the conidial solution (dilution 1:10) was applied by nebulisation on both sides of each slice at a rate of approximately 1 ml (ca. 10⁴ spores) per bread slice. Each slice was then packed in a plastic bag and heat sealed, during which procedure a small slot was left open and a tip of a transfer pipette was inserted to ensure comparable aerobic conditions in each bag. Bags were incubated at room temperature and examined for mould growth during a ten-day storage period. A series of ten slices was inoculated. Mould growth was quantified as being the number of slice surfaces, i.e. both front and rear of slice, manifesting air mycelia.

### Lean bread and Japanese Rolls shelf life tests

*L. amylovorus* FST 2.11 was investigated in form of sourdough for the potential to increase the shelf life of lean bread and Japanese rolls. For both experiments, *L. amylovorus* sourdough fermented for 48 h at 30°C was added at 40% level (20% flour replacement), and the resulting bread/Japanese roll was challenged against contaminants present in an industrial bakery, including *Penicillum aethiopicum* and *A. niger.* Briefly, bread/rolls were exposed to the bakery air for about 10min, packaged, stored at room temperature and mould growth was observed daily. The shelf life of bread containing 20% sourdough fermented by *L. amylovorus* was compared to that of standard bread (not containing sourdough) as well as bread containing 0.3% calcium propionate (maximum level allowed of chemical preservatives; calcium propionate is used as a preservative in a variety of products).

### RESULTS

### Isolation of sourdough LAB

Bacteriological culturing of spontaneously fermented sourdoughs revealed LAB cell counts of ca. 3 x 10⁹ CFU/g sourdough. From the predominant LAB, isolates were subcultured. Analysis of the partial 16S rDNA identified the following species among the predominant LAB of gluten-free sourdoughs: *L. amylovorus, Lactobacillus brevis, Lactobacillus johnsonii, L. plantarum, Lactobacillus reuteri, L. sanfranciscensis,* and *Weissella cibaria.*

### In vitro antimicrobial activity

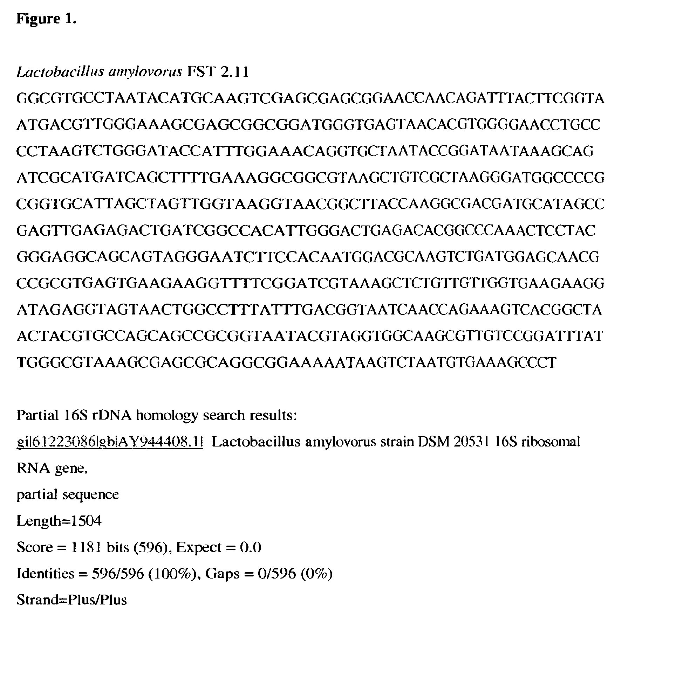

The sourdough LAB were tested *in vitro* for antimicrobial activity against *A. fumigatus* (data not shown). Among the different strains tested, one strain, i.e. *L. amylovorus* FST 2.11 (DSM 19280; see Fig. 1 for 16S rDNA homology results), was found to be highly inhibitory against *A. fumigatus* and was therefore selected for further investigations. The inhibitory spectrum of *L. amylovorus* FST 2.11 against several bacteria as well as fungi was investigated using an agar diffusion assay (Dal Bello et al., 2007). Results are summarised in Table II. The antifungal strain *L. plantarum* FST 1.7 (Dal Bello et al., 2007) was included in the screening together with the negative control *L. sanfranciscensis* LTH 2581. Most of the bacteria tested were inhibited by all selected LAB, however, only the strains *L. amylovorus* FST 2.11 and *L. plantarum* FST 1.7 inhibited the growth of common moulds *in vitro.* None of the tested LAB was able to inhibit the growth of *P. roqueforti in vitro.*

### Antifungal compounds produced by L. amylovorus FST 2.11

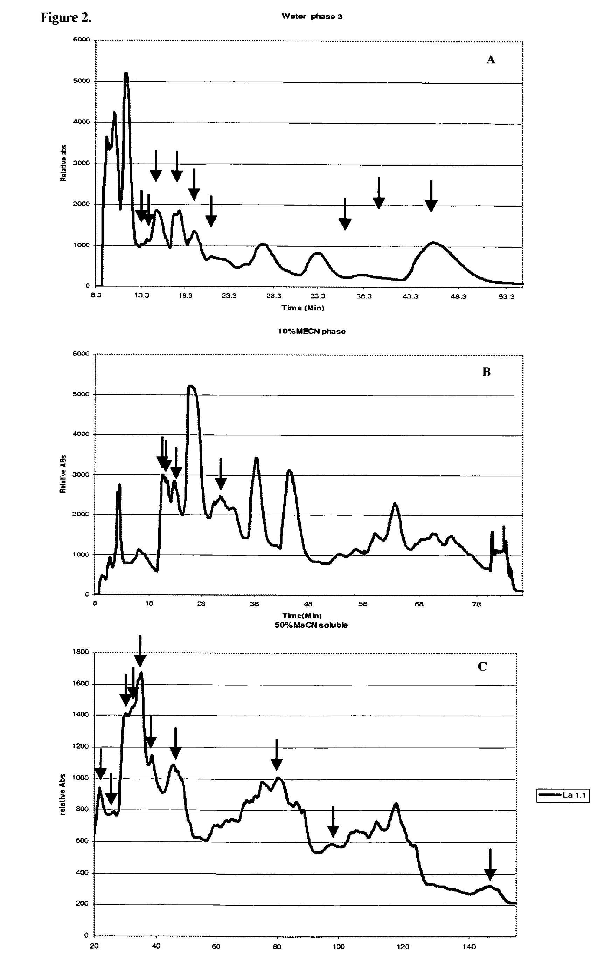

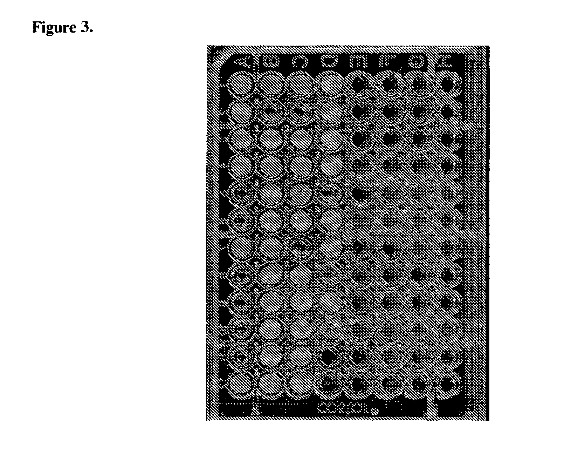

Isolation of antifungal compounds produced by *L. amylovorus* FST 2.11 was performed using a spore germination bioassay. Both the hydrophilic and the hydrophobic phases were found to contain compounds showing strong inhibitory activity against spores of *A. fumigatus* (Table III). Compounds present in the hydrophilic and hydrophobic phases were separated using HPLC (see Fig. 2 as example). Overall, 27 antifungal compounds were isolated using the bioassay (see Fig. 3 as example), and 17 compounds were identified using NRMS, MS and GC (Table III).

### Microbiological analysis of wheat sourdoughs

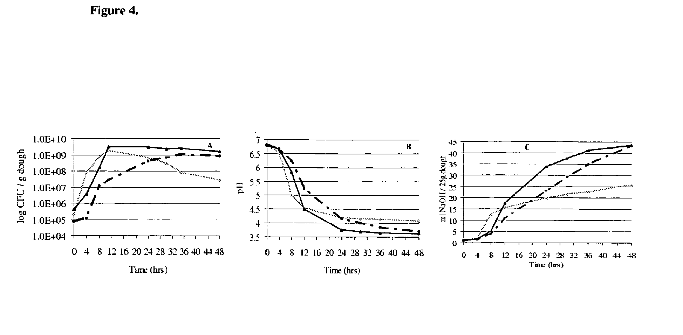

LAB growths during wheat sourdough fermentation are shown in Fig. 4. All the tested strains showed microbiological values (CFU, pH, TTA) typical of wheat sourdough fermentations (Meroth et al., 2003). For all the investigated sourdoughs, LAB cell counts of about 2-3 x 10⁹ CFU/g sourdough were recovered. Identification of the isolates belonging to the predominant microbiota after 48 hrs of fermentation revealed that each of the strain persisted throughout the fermentation, thus indicating that the selected strains are suitable for wheat sourdough production.

### Dough and bread analysis

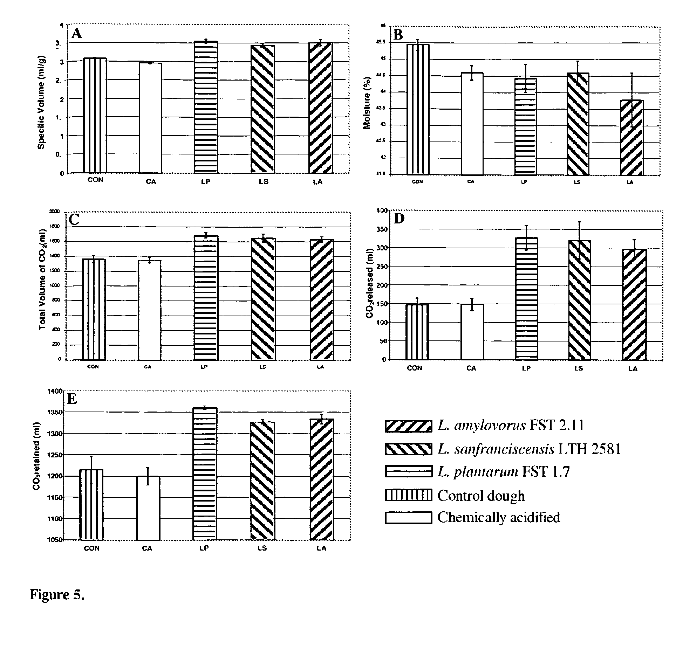

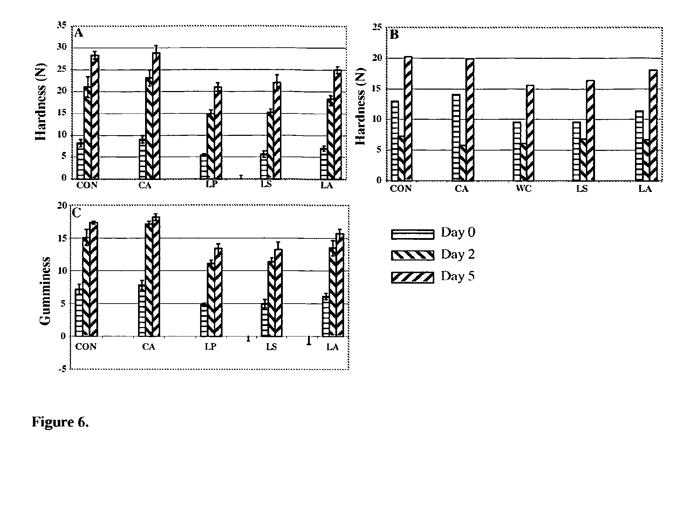

The suitability of *L. amylovorus* FST 2.11 as starter for sourdough fermentation, and thus sourdough bread production, was evaluated using a series of tests, i.e. specific volume, moisture content, CO₂ produced/released, as well as hardness and rate of staling (Fig. 5 and 6). As controls, a chemically acidified dough as well as a non fermented dough were used. Taking into consideration all investigated parameters, the bread containing sourdough fermented by *L. amylovorus* FST 2.11 did not significantly differ from bread containing sourdough fermented by either *L. plantarum* FST 1.7 or the traditional sourdough-starter *L. sanfranciscensis,* thus clearly showing the suitability of strain DSM 19280 as starter for wheat sourdough production. All wheat sourdough breads showed improvement quality when compared to wheat bread or wheat bread produced using a chemically acidified dough. Addition of sourdough resulted in an increase in the specific volume of the bread (Fig. 5A), and thus of the bread volume and density, both being important parameters for consumer acceptability. Finding higher moisture content (Fig. 5B) in the control breads compared to the sourdough breads can also be explained by the biological acidification. During fermentation the drop in pH leads to increased enzyme activity as well as protein denaturation, and thus to a softening of the protein network. This in turn results in a higher release of water which can evaporate off during the baking process. Furthermore, proteolytic activity of the bacteria also leads to degradation of the protein network and thus to a higher loss of water during the baking process. The results concerning total, released and retained CO₂ are presented in Fig. 5C, 5D and 5E, respectively. Overall, the addition of sourdough resulted in higher CO₂ production. This can be due to fermentation by heterofermentative LAB and/or by endogenous yeasts. Furthermore, the sugar and amino acids released by the bacteria can be easily used by the added yeasts, thus increasing the gas production. However, due to the biological acidification, most of the proteins have been degraded and the structure is weakened, as can be seen by the higher gas release of the sourdough samples when compared to the controls (Fig. 5D). This weakening of the structure is however masked by the increased amount of gas produced, as can be seen in Fig. 5E.

Analysis of the hardness and rate of staling revealed that addition of sourdough, independent from the inoculated strain, resulted in softer bread with a delayed rate of staling, when compared to the control breads (Fig. 6A and 6B). The initial softening of the bread is a result of the sourdough enzymatic activity, which is responsible for the starch and protein breakdown. However, as we progress to day 2 and day 5 the hardness of the bread is dominated by the starch portion of the bread. Figure 6C depicts the gumminess of the breads, which is a key parameter (mouth-feeling) for the consumer. Gumminess is perceived as a negative trait in bread. As shown, the addition of sourdough leads to a reduction in gumminess and so will improve consumer acceptability over the 5 days of storage. Again, *L. amylovorus* sourdough bread was not significantly different from *L. sanfranciscensis* sourdough bread, thus indicating that the addition of sourdough fermented by strain FST 2.11 leads to the positive effects that are traditionally associated with the use of sourdough.

### Bread challenge

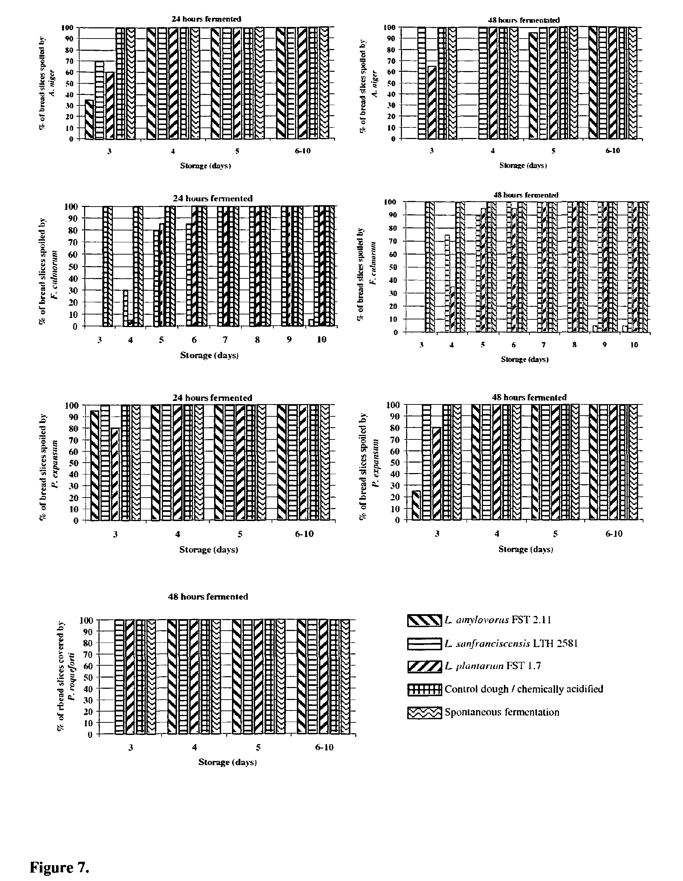

In addition to the evaluation of its antimicrobial activity *in vitro*, *L. amylovorus* FST 2.11 was investigated in form of sourdough for the potential to increase the shelf life of wheat bread (Fig. 7). *L. amylovorus* sourdough fermented for 24 or 48 h was added at 20% level, and the resulting bread was challenged with spores of *A. niger, F. culmorum, P. expansum* or *P. roqueforti.* Results were compared to those obtained using sourdough fermented by the antifungal *L. plantarum* FST 1.7, the non antifungal *L. sanfranciscensis* LTH 2581 or by the endogenous wheat flour biota (spontaneous fermentation). Twenty-four or 48 h fermented sourdough delayed the mould growth in a strain- and fungus- dependent manner (Fig. 7). Overall, a retarded mould growth was observed when bread contained sourdough, but the addition of sourdough fermented by the antifungal strains resulted in a higher delay in mould growth. *L. amylovorus* FST 2.11 revealed to be by far the strongest antifungal strain, especially when 48h fermented sourdough was used. Compared to the other investigated breads, addition of *L. amylovorus* fermented sourdough retarded up to 2 days the growth of *A. nigger,* up to 7 days the growth of *F. culmorum,* and up to one day the spoilage of *P. expansion* or *P. roqueforti.* The same pH and TTA was measured in the two antifungal sourdoughs, i.e. sourdough fermented by *L. amylovirus* and sourdough fermented by *L. plantarum.* However, the increase in shelf life of wheat sourdough bread was significantly higher when fermentation was performed by *L. amylovorus,* thus indicating that strain FST 2.11 produces unique compounds which are responsible for this dramatic inhibition of mould growth.

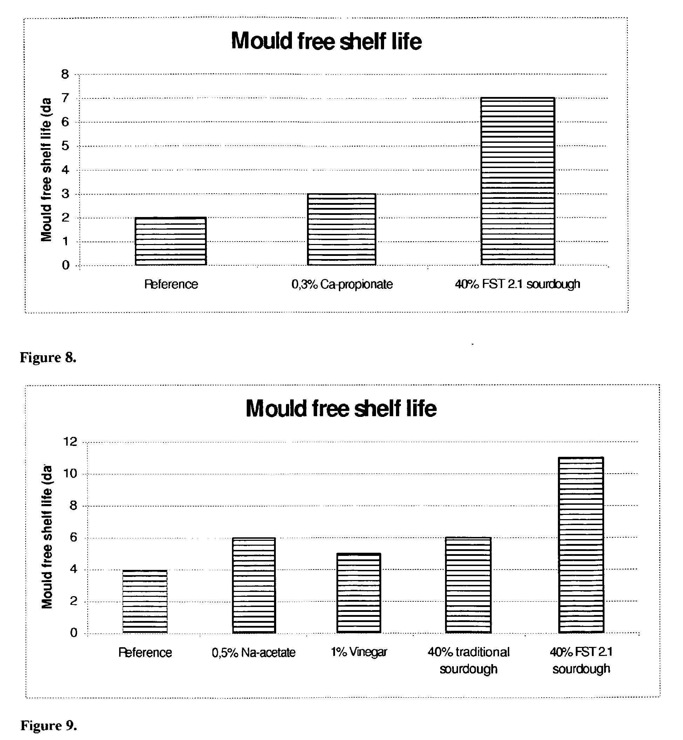

*L. amy*/*ovorus* FST 2.11 was also investigated in form of sourdough for the potential to increase the shelf life of lean bread and Japanese rolls (Fig. 8 and 9). Results obtained from the Japanese rolls containing 20% sourdough fermented by *L. amylovorus* were compared with those of Japanese rolls containing 20% traditional sourdough, 1% vinegar, 0.5% Naacetate, or no additives (standard rolls). The traditional sourdough was prepared with *L. brevis* (Fig. 9). For both bread and Japanese rolls, a significant increase in the shelf life of products was obtained only when *L. amylovorus* sourdough was used. In particular, the presence of 20% sourdough fermented by *L. amylovorus* resulted in products with a longer shelf life than those containing maximum levels of chemical additives. Thus, *L. amylovorus* FST 2.11 revealed to be by far the strongest antifungal strain.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### Literature Cited

1. Arbeitsgemeinschaft Getreideforschung e.V. (AGF), 1994. Standard-Methoden für Getreide, Mehl und Brot. 7. überarbeitete und erweiterte Auflage. 7th ed. Verlag Moritz Schäfer: Detmold, Germany.
2. Clarke, C.I., Schober, T.J., Arendt, E.K., 2002. Cereal Chemistry 79, 640-647.
3. Corsetti, A., Gobbetti, M., De Marco, B., Balestrieri, F., Paoletti, F., Russi, L., Rossi, J., 2000. Journal of Agricultural Food Chemistry 48, 3044-3051.
4. Crowley, P., Schober, T.J., Clarke, C.I, Arendt, E.K., 2002. European Food Research and Technology 214, 489-496.
5. Dal Bello F., C.I. Clarke, L.A.M. Ryan, H. Ulmer, K. Ström, J. Sjögren, D. van Sinderen, J. Schnürer, and E.K. Arendt. 2007. Journal of Cereal Science, 45: 309-318.
6. Gray, J.A., Bemiller, J.N., 2003. Comprehensive Reviews in Food Science and Food Safety 2, 1-21.
7. Graz, M., A. Hunt, H. Jamie, G. Grant, Milne, P., 1999. Pharmazie 54, 772-775.
8. Legan, J.D., 1993. International biodeterioration and biodegradation 32, 33-53.
9. Lindgren, S.E., Dobrogosz, W.J., 1990. FEMS Microbiology Reviews 87, 149-163.
10. Magnusson, J., Schnürer, J., 2001. Applied and Environmental Microbiology 67, 1-5.
11. Magnusson, J., Ström, K, Roos, S., Sjögren, J., Schnürer, J., 2003. FEMS Microbiology Letters 219, 129-135.
12. Meroth, C., Walter, J., Hertel, C., Brandt, M.J., Hammes, W.P., 2003. Applied and Environmental Microbiology 69, 475-482.
13. Niku-Paavola, M.L., Laitila, A., Mattila-Sandholm, T., Haikara, A., 1999. Applied Microbiology 86, 29-35.
14. Okkers, D.J., Dicks, L.M.T., Silvester, M., Joubert, J.J., Odendaal, H.J., 1999. Journal of Applied Microbiology 87, 726-734.
15. Pateras, I.M.C., 1998. Bread spoilage and staling. In: Technology of breadmaking S.P. Cauvain, and L.S. Young Eds. Blackie Academic and Professional: London, pp. 240-261.
16. Poute, J.G., Tsen, C.C., 1987. Bakery products. In: L.R. Beuchat (Ed.) Food and beverage mycology. 2nd ed., AVI Van Nostrand Reinhold, NewYork, pp. 233-267.
17. Röcken, W., Voysey, P.A., 1995. Journal of Applied Bacteriology 79, 38S-48S.
18. Röcken, W., 1996. Advances in Food Science 18, 212-216.
19. Stiles, M.E., 1996. Antonie van Leeuwenhoek 70, 331-345.
20. Ström, K., Sjörgren, J., Broberg, A., Schnürer, J., 2002. Applied and Environmental Microbiology 68, 4322-4327.
21. Ström, K, Schnürer, J., Melin, P., 2005. FEMS Microbiology Letters 246, 119-24.

**Table I. Gluten-free sourdough composition.**

| **Sourdough** | Weight (grams) |
|---|---|
| Brown rice flour | 200 |
| Buckwheat (jade) | 50 |
| Soya flour | 20 |
| Potato starch | 100 |
| Water | 370 |

**Table II. In vitro inhibitory activity of L. amylovorus DSM 19280 against common moulds and spoilage bacteria.**

| **Bacteria** | *L. plantarum* FST 1.7 | *L. sanfranciscensis* LTH 2581 | *L. amylovorus* FST 2.11 |
|---|---|---|---|
| *Bacillus subtilis* FST 2.2 | ++ | + | + |
| *Citrobacter freundi* FST 2.7 | ++++ | +++ | ++ |
| *Enterococcus faecalis* FST 2.8 | + | + | ++ |
| *Escherichia coli* FST 2.3 | +++ | +++ | +++ |
| *Listeria innocua* FST 2.5 | ++++ | ++++ | ++ |
| *Micrococcus luteus* FST 2.10 | ++++ | +++ | ++ |
| *Proteus vulgaris* FST 2.12 | +++ | ++ | ++ |
| *Staphylococcus aureus* FST 2.4 | +++ | ++ | ++ |
| *S. aureus* TMW 2.127 | ++++ | +++ | ++ |

| **Moulds** | | | |
|---|---|---|---|
| *Aspergillus niger* FST 4.21 | ++ | - | ++ |
| *Fusarium graminearum* FST 4.0122 | ++ | - | ++ |
| *F. graminearum* TMW 4.0208 | +++ | + | ++ |
| *F. culmorum* TMW 4.0754 | +++ | - | ++ |
| *Fusarium oxysporum* FST 4.03 | ++ | - | ++ |
| *Penicillium roqueforti* TMW 4.1599 | - | - | - |

**Table III. Antifungal compounds produced in nitro by L. amylovorus FST 2.11 showing strong inhibitory activity against A. fumigatus.**

| **Code** | **Compound** | **Chemical formula** | **MW** | **Fragments (MW)** | **Increase in Shelf life vs. control (drays)**** |
|---|---|---|---|---|---|
| 1 | Glucuronic acid* | C₆H₁₀O₇ | 194.14 | 105.1,91.1 | >10 |
| 2 | Cytidine* | C₉H₁₃N₃O₅ | 243.22 | 109.1, 91.1 | 1 |
| 3 | Deoxycytidine*,*** | C₉H₁₃N₃O₄ | 227.22 | 183.0,93.1 | 1 |
| 4 | Sodium decanoate* | C₁₀H₁₉O₂Na | 194.25 | 153.3, 103.0 | >10 |
| 5 | Hydrocinnamic acid* | C₉H₁₀O₂ | 150.17 | 105.1 | >10 |
| 6 | Phenyllactic acid | C₉H₁₀O₃ | 166.17 | 149.0, 119.8 | >10 |
| 7 | OH- Phenyllactic acid* | C₉H₁₀O₄ | 182.17 | 163.1, 135.2 | >10 |
| 8 | Coumaric acid* | C₉H₈O₃ | 164.16 | 119.2 | >10 |
| 9 | Methylcinnamic acid* | C₁₀H₁₀O₂ | 162.19 | 147.1, 117.1 | >10 |
| 10 | Salicylic acid* | C₇H₆O₃ | 136.12 | 93.2 | >10 |
| | | | | | |
| 11 | Cyclo(His - Pro) | C₁₁H₁₄N₄O₂ | 234.26 | 207.1, 166.0, 110.1 | 2 |
| 12 | Cyclo(Pro - Pro) | C₁₀H₁₄N₂O₂ | 194.23 | 151.2,70 | 4 |
| 13 | Cyclo(Met - Pyro)*** | C₁₀H₁₆N₂O₂S | 228.3 | 181.2, | 3 |
| 14 | Cyclo(Tyr - Pro) | C₁₄H₁₆N₂O₃ | 260.3 | 233.0, 136.1 | 3 |
| 15 | Cyclo(Leu - Pro) | C₁₁H₁₈N₂O₂ | 210.13 | 183.2, 155.0, 86 | 3 |
| 16 | Lactic acid | C₃H₆O₃ | 90.08 | - | 1 |
| 17 | Acetic acid | C₂H₄O₂ | 60.05 | - | 1 |

| | | | | | |
|---|---|---|---|---|---|
| * Electron spray ionization in the negative mode was used for MS/MS fragmentation of molecules on Q TOF and LCQ LC- MS ** 50mg/g of each compound and was evaluated over 12 days for the ability to stop the outgrowth of 10⁴/ml of *A. fumigatus* spores. Controls were spoiled after 2 days of growth *** Previously unreported antimicrobial compound | | | | | |

## Claims

1. A strain of *Lactobacillus amylovorus* designated FST 2.11 as deposited under the accession no DSM 19280 on 13^{th} April 2007 in the DSMZ depository.

2. A starter culture for bread including low salt bread, or cereal-based products comprising a strain as claimed in Claim 1.

3. A food ingredient comprising a strain as claimed in Claim 1.

4. An antifungal agent active against spoilage moulds, comprising a strain as claimed in Claim 1.

5. A method of production of fermented cereal products or breads including low salt bread, comprising addition of the strain of Claim 1 to the cereals or bread starting materials.

6. A pharmaceutical composition comprising a strain as claimed in Claim 1 and a pharmaceutically acceptable carrier or excipient.

## Patentansprüche

1. Stamm von *Lactobacillus amylovorus* mit der Bezeichnung FST 2.11, wie unter der Zugangsnummer DSM 19280 am 13. April 2007 in der DSMZ-Hinterlegungsstelle hinterlegt.

2. Starterkultur für Brot, das salzarmes Brot einschließt, oder getreidebasierte Produkte, die einen Stamm nach Anspruch 1 umfasst.

3. Nahrungsbestandteil, der einen Stamm nach Anspruch 1 umfasst.

4. Antimykotikum, das gegen den Verderb bewirkende Schimmelpilze aktiv ist, das einen Stamm nach Anspruch 1 umfasst.

5. Verfahren zur Herstellung von fermentierten Getreideprodukten oder Broten, die salzarmes Brot einschließen, das die Zugabe des Stamms nach Anspruch 1 zum Getreide oder zu den Brotausgangsstoffen umfasst.

6. Pharmazeutische Zusammensetzung, die einen Stamm nach Anspruch 1 und einen pharmazeutisch verträglichen Träger oder Hilfsstoff umfasst.

## Revendications

1. Souche de *Lactobacillus amylovorus* désignée sous le nom de FST 2.11 et telle que déposée auprès de la DSMZ sous le numéro d'entrée DSM 19280 le 13 avril 2007.

2. Culture d'amorçage destinée à la fabrication de pains, y compris de pains à faible teneur en sel, ou de produits à base de céréales, comprenant une souche selon la revendication 1.

3. Ingrédient alimentaire comprenant une souche selon la revendication 1.

4. Agent antifongique actif contre les moisissures indésirables comprenant une souche selon la revendication 1.

5. Méthode de production de produits céréaliers ou de pains fermentés, y compris de pains à faible teneur en sel, comprenant l'addition de la souche de la revendication 1 aux matières premières de départ des produits céréaliers ou des pains.

6. Composition pharmaceutique comprenant une souche selon la revendication 1 et un véhicule ou excipient pharmaceutiquement acceptable.
